# EUROPÄISCHE PATENTSCHRIFT

(11) **EP 2 807 246 B1**
(45) Veröffentlichungstag und Bekanntmachung des Hinweises auf die Patenterteilung: **27.02.2019**
(21) Anmeldenummer: 13701248.0
(22) Anmeldetag: 23.01.2013
(51) Int. Cl.: C12M 3/00, C12M 1/12

(54) **BIO- UND MEDIZINTECHNISCHES BAUKASTENSYSTEM**
BIOENGINEERING AND MEDICAL MODULAR SYSTEM
SYSTÈME MODULAIRE BIOTECHNIQUE ET DE TECHNIQUE MÉDICALE

(30) Priorität: 23.01.2012 DE 102012200938
(43) Veröffentlichungstag der Anmeldung: 03.12.2014
(73) Patentinhaber: ALPHA PLAN GmbH, 01454 Radeberg (DE)
(72) Erfinder: NAGELS, Hans, 37120 Bovenden (DE)
(74) Vertreter: Kailuweit & Uhlemann Patentanwälte Partnerschaft mbB
(86) Internationale Anmeldenummer: PCT/EP2013/051230
(87) Internationale Veröffentlichungsnummer: WO 2013/110651

(56) Entgegenhaltungen:
- WO-A1-2006/119622
- WO-A2-2006/122089
- US-A1- 2008 213 894
- US-A1- 2010 105 138

## Beschreibung

Die Erfindung betrifft bio- und medizintechnische Baukastensysteme umfassend eine in sich geschlossenen Einrichtung zur Beeinflussung von biologischen Medien, Zellen, Gewebe und gewebeähnlicher Strukturen als Objekte.

Bioreaktoren sind entsprechend ihrer Anforderungen und Betriebsweisen bekannt.

Durch die Druckschrift DE 40 06 382 A1 ist ein Bioreaktor zur Durchführung mikrobieller Prozesse am Labor- und Technikumsmaßstab und zur Entwicklung prozessangepasster technischer Bioreaktoren bekannt. Dazu besteht dieser aus verschiedenen miteinander verbindbaren Funktionsmodulen und Adaptern. Die Reihenfolge der Funktionsmodule und Adapter untereinander ist beliebig wählbar.

Die Druckschrift DE 41 18 882 A1 beinhaltet ein Modulsystem zum Aufbau von Bioreaktoren, die innerhalb eines bestimmten Bereichs abgestufte Durchmesser aufweisen können. Die Druckschriften sind auf Bioreaktoren beschränkt.

Durch die Druckschrift US 2002 0 055 166 A1 ist ein Gestell mit mindestens zwei Kassetten zur Verwendung in einem Inkubator bekannt. Die Kassetten sind jeweils als Gehäuse für einen vertikalen Einsatz ausgeführt. Alle Elemente des Systems sind im Gehäuse angeordnet.

Aus der Druckschrift US 5 202 254 A ist eine Vorrichtung zur Beeinflussung von biologischen Medien, Zellen oder Geweben bekannt. Diese weist einen Behälter für flüssiges Medium, eine Fördereinrichtung für das Medium, Verbindungselemente, Gasaustauschfilter und einen damit verbundenen Hohlfaserbioreaktor auf. Ein Basiskörper wird nicht beschrieben.

Die Druckschrift WO 2006/122089 A2 umfasst eine Schale für die zum Betrieb notwendigen Instrumente/Geräte und eine Anordnung, die eine Kultivierungseinrichtung und Vorratsbehälter aufweist. Letztere ist separat auf die Schale aufgesetzt. Im Betriebszustand werden die Instrumente/Geräte an die auf der Anordnung platzierten Einrichtungen gekoppelt. Diese sind auf und nicht in der Schale platziert. Es ist damit eine räumliche Trennung zwischen der Schale und der Anordnung vorhanden. In der Schale befinden sich nur die Instrumente/Geräte. Grundlage des Systems ist der, dass die Anordnung mit der Kultivierungseinrichtung und den Behältern austauschbar ist. Bei Platzierung einer neuen Anordnung ist die Kopplung zu den Instrumenten/Geräten der Schale wieder sichergestellt. Es handelt sich also nicht um ein Baukastensystem. Die Anordnung der Kultivierungseinrichtung und der Vorratsbehälter ist durch die in der Schale platzierten Instrumente/Geräte vorgegeben.

Ein weiterer Nachteil besteht darin, dass sich die Kultivierungseinrichtung und die Behälter nicht in der Schale befinden. Bei Leckagen wird das entsprechende Medium nicht durch die Schale aufgefangen.

Der im Patentanspruch 1 angegebenen Erfindung liegt die Aufgabe zugrunde, ein System von Elementen so bereitzustellen, dass nutzerspezifische Einrichtungen zur Beeinflussung von biologischen Medien, Zellen, Gewebe und gewebeähnlicher Strukturen als Objekte einfach und ökonomisch günstig realisierbar sind.

Diese Aufgabe wird mit den im Patentanspruch 1 aufgeführten Merkmalen gelöst.

Die medizintechnische Baukastensysteme umfassend eine in sich geschlossenen Einrichtung zur Beeinflussung von biologischen Medien, Zellen, Gewebe und gewebeähnlicher Strukturen als Objekte zeichnen sich insbesondere dadurch aus, dass die nutzerspezifischen Einrichtungen zur Beeinflussung von biologischen Medien, Organismen oder Teilen von Organismen einfach und ökonomisch günstig realisierbar sind.

Dazu ist ein wannenförmiger und horizontal angeordneter Basiskörper zur Platzierung von miteinander verbindbaren Elementen vorhanden.

Weiterhin sind wenigstens ein Behälter für ein flüssiges Medium, mindestens eine Fördereinrichtung für flüssiges Medium an einer Wand am oder im wannenförmigen Basiskörper oder als Bestandteil einer Wand des Basiskörpers, Verbindungselemente, Filter zur Be- und Entgasung, mindestens ein Filter mit einem Anschlusselement für Medium, wenigstens ein Sensor und/oder eine Einrichtung zur Bestimmung wenigstens eines Stoffes des Mediums und/oder im Medium, Elemente für eine Verbindung mit wenigstens einem Bioreaktor und/oder mindestens einer Begasungseinheit weitere Bestandteile des Baukastensystems.

Das Antriebsmittel oder die Energiequelle für das Antriebsmittel der Fördereinrichtung ist von außen lösbar anschließbar.

Der Bioreaktor oder die Begasungseinheit oder die Kombination mit wenigstens einem Bioreaktor und mindestens einer Begasungseinheit ist ein weiteres Element und damit ein weiterer Bestandteil des medizinischen Baukastensystems.

Weiterhin sind sowohl ein Bereich für wenigstens einen Bioreaktor, mindestens eine Begasungseinheit oder eine Kombination mit wenigstens einem Bioreaktor und mindestens einer Begasungseinheit als auch ein Bereich für den Behälter für ein flüssiges Medium vorhanden, die durch wenigstens eine Zwischenwand voneinander getrennt sind.

Weiterhin ist eine Aufnahmestation für den wannenförmigen Basiskörper ein Bestandteil des Baukastensystems. Bei Platzierung des wannenförmigen Basiskörpers in der Aufnahmestation ist das Antriebsmittel oder die Energiequelle für das Antriebsmittel mit der Fördereinrichtung verbunden. Darüber hinaus ist das Antriebsmittel für die Fördereinrichtung und entweder der Sensor und/oder die Einrichtung zur Bestimmung wenigstens eines Stoffes des Mediums und/oder im Medium mit einer Steuereinrichtung als Bestandteil der Aufnahmestation verbunden. Die Steuereinrichtung kann ein bekanntes Datenverarbeitungssystem in Form eines Mikrocontrollers sein. Mit einer entsprechenden Software kann der Ablauf zur Beeinflussung von biologischen Medien, Organismen oder Teilen von Organismen als Objekte entsprechend der vorhandenen Eigenschaften gesteuert werden.

Verbindungselemente können dabei günstigerweise gerade Rohrstücke, gebogene Rohrstücke, Schlauchstücke, Verbinder und/oder Verteiler sein. Letztere weisen beispielsweise eine T- oder Y-Form auf. Die Abmessungen sind dabei vorteilhafterweise so ausgeführt, dass die Endenbereiche der Schlauchstücke einfach auf die jeweilig anderen Verbindungselemente dichtend aufschiebbar sind. Damit lassen sich einfach Verbindungen der einzelnen Elemente realisieren. Darüber hinaus kann mindestens ein Wechselventil und/oder ein Rückschlagventil ein Element und/oder Elemente sein.

Der wannenförmige Basiskörper besitzt wenigstens eine Zwischenwand. Die Räume für den Behälter mit dem flüssigen Medium und für den Bioreaktor oder die Begasungseinheit oder der Kombination davon sind durch die Zwischenwand vorteilhafterweise voneinander getrennt. Weiterhin erhöht sich die mechanische Stabilität des Basiskörpers.

Die Böden der Räume und damit die Bereiche einerseits für den Behälter mit dem flüssigen Medium und andererseits für den Bioreaktor oder die Begasungseinheit oder der Kombination davon können in einer Ebene oder in unterschiedlichen Ebenen ausgebildet sein. Im letzteren Fall weist der wannenförmige Basiskörper eine Stufe im Boden auf. In einer weiteren Variante kann der Boden des Behälters von der Zwischenwand nach außen hinabfallend ausgebildet sein, so dass eventuell aus dem Behälter auslaufendes Medium in Richtung der der Zwischenwand gegenüberliegenden Außenwand läuft und sich dort ansammelt. Die Gefahr, dass Medium aus dem Behälter in den Raum mit dem Bioreaktor oder der Begasungseinheit oder der Kombination davon gelangt, ist damit weiter vermindert.

Vorteilhafterweise kann der wannenförmige Basiskörper Befestigungselemente für Elemente des Baukastensystems besitzen. Das sind insbesondere beabstandet angeordnete Wandbereiche, zwischen die Bereiche von Schlauchstücken als Verbindungselemente klemmbar sind. Weiterhin sind das Öffnungen oder Ösen für Befestigungseinrichtungen.

Die Fördereinrichtung für flüssiges Medium ist so angeordnet, dass ein Antriebsmittel für die Fördereinrichtung oder die Energiequelle für ein mit der Fördereinrichtung gekoppeltes Antriebsmittel von außen lösbar anschließbar ist. Die Fördereinrichtung und das Antriebsmittel sind die Bestandteile einer bekannten Pumpe. Diese Bestandteile sind voneinander lösbar ausgebildet. Elektrische Zuleitungen in den Basiskörper sind zum Betrieb der Pumpe nicht notwendig. Dadurch ist der elektrisch zu betreibende Teil des Baukastensystems von der Einrichtung zur Beeinflussung von biologischen Medien, Zellen, Gewebe oder gewebeähnlichen Strukturen getrennt. Elektrischen Strom führende Einrichtungen werden im Basiskörper vermieden.

Wenigstens ein Sensor und/oder eine Einrichtung zur Bestimmung wenigstens eines Stoffes des Mediums und/oder im Medium ist/sind ein Element oder Elemente. Der Sensor ist dazu insbesondere ein pH-Wert-Sensor oder ein Sauerstoffsensor. Die Einrichtung zur Bestimmung eines Stoffes ist beispielsweise eine optische Einrichtung, wobei über optisch detektierbare Veränderungen des Mediums die Zusammensetzung oder einzelne Stoffe des Mediums bestimmbar sind. Strahlungsquellen sind dazu bekannte Quellen für elektromagnetische Strahlen.

Die Fördereinrichtung und das Antriebsmittel sind die Bestandteile einer bekannten Pumpe. Diese Bestandteile sind voneinander lösbar ausgebildet.

Das Antriebsmittel kann dazu ein bekannter Elektromotor sein. Der Rotor des Elektromotors ist in oder mit der Fördereinrichtung lose platziert. Die Bewegungsübertragung kann dabei günstigerweise durch eine besondere Form sichergestellt sein. Im einfachsten Fall ist der Rotor bereichsweise abgeflacht oder im Querschnitt mehreckig ausgebildet. Die Fördereinrichtung kann dabei eine Flügelzelle, Zahnräder, eine Schraubenspindel oder Drehkolben aufweisen.

Die Fördereinrichtung kann auch eine Membran sein. Die Membran kann mittels eines mechanisch oder elektromagnetisch wirkenden Antriebsmittels ausgelenkt werden. Bei einer Variante mit einer mechanischen Auslenkung kann das über ein schwingendes Antriebsmittel beispielsweise in Form eines Piezoantriebs oder eines an einen Elektromotor gekoppelten Exzenters erfolgen. Bei einer Variante mit einer elektromagnetisch hervorgerufenen Auslenkung kann das Antriebsmittel ein Schwinganker sein.

Vorteilhafte Ausgestaltungen der Erfindung sind in den Patentansprüchen 2 bis 11 angegeben.

Der Bioreaktor weist nach der Weiterbildung des Patentanspruchs 2 ein Gehäuse mit einem Bündel nach außen offener Hohlfasermembranen und kappenförmigen Endstücken auf. Das Gehäuse weist einen Rohrabschnitt mit Anschlüssen für den Raum mit den Hohlfasermembranen auf. Die Endenbereiche der Hohlfasermembranen befinden sich in Böden aus einem Kunststoff. Die Endstücke weisen jeweils einen Anschluss für die Hohlräume der Hohlfasermembranen auf. Das ist eine einfache und ökonomisch günstige Realisierung des Bioreaktors. Das Bündel mit den Hohlfasermembranen wird im Rohr platziert. Dabei sind die Hohlfasermembranen länger als der Rohrabschnitt. In die Endenbereiche des Rohrstücks mit den Hohlfasermembranen wird ein Kunststoff eingebracht, der nach dem Aushärten die Böden des Gehäuses bildet. Der Kunststoff dichtet dabei gleichzeitig den die Hohlfasermembranen umgebenden Raum und den Innenraum des Rohrabschnitts nach außen ab. Die überstehenden Hohlfasermembranen werden danach einfach abgetrennt, so dass die Hohlräume der Hohlfasermembranen von außen zugänglich sind. Auf die Endenbereiche des Rohrabschnitts werden die Endstücke aufgeschoben. Letztere sind so ausgebildet, dass diese und die Böden jeweils einen Raum begrenzen. Anschlüsse des Rohrabschnitts und der Endstücke gewährleisten den Zugang der jeweiligen Medien.

Das Bündel von Hohlfasermembranen kann im Querschnitt kreisförmig, oval, mehreckig, rechteckig mit abgerundeten Ecken oder rechteckförmig mit runden Seiten ausgebildet sein.

Der Bioreaktor weist nach der Weiterbildung des Patentanspruchs 3 einen wannenförmigen Grundkörper mit jeweils wenigstens einem Einlass und einem Auslass in einer der Seitenwände für das Medium und einem von dem wannenförmigen Grundkörper entfernbaren Deckel ohne oder mit mindestens einem Septum auf. Das ist eine einfache und ökonomisch günstige Realisierung eines Bioreaktors. Weiterhin ist ein solcher universell einsetzbar, wobei die verschiedensten spezifischen Einbauten im Bioreaktor platzierbar sind. Dieser kann damit leicht entsprechend der zu beeinflussenden Objekte ausgestaltet werden, so dass ein derartiger Bioreaktor universell verwendbar ist.

Der wannenförmige Grundkörper und der Deckel können vorteilhafterweise lösbar dicht miteinander verbunden sein, so dass die Sterilität gewährleistet ist.

Der Bioreaktor weist nach der Weiterbildung des Patentanspruchs 4 einen wannenförmigen Grundkörper mit jeweils wenigstens einem Einlass und einem Auslass in einer der Seitenwände für das Medium, mindestens eine Barriere im wannenförmigen Grundkörper oder als Bestandteil des wannenförmigen Grundkörpers, einen plattenförmigen Träger mit mindestens einem eine Membran aufweisenden Behältnis für das wenigstens eine Objekt in oder als Bestandteil des plattenförmigen Trägers im wannenförmigen Grundkörper und einen von dem wannenförmigen Grundkörper entfernbaren Deckel mit mindestens einem Septum auf.

Der Einlass und der Auslass sind dabei so angeordnet, dass die Höhe der Barriere die Höhe der Oberfläche des Mediums im wannenförmigen Grundkörper bestimmt. Darüber hinaus befindet sich wenigstens die Membran des Behältnisses im Medium des wannenförmigen Grundkörpers.

Der Boden des wannenförmigen Grundkörpers kann vorteilhafterweise eine korrespondierend zum Behältnis angeordnete Vertiefung aufweisen, so dass zwischen dem Behältnis für das wenigstens eine Objekt und der Vertiefung ein Abstand für das Nährmedium vorhanden ist. Weiterhin ist die Barriere höher als die Ebene des Bodens mit der Vertiefung.

Der Raum für das Nährmedium ist dadurch in der Einrichtung gleichbleibend begrenzt. Die Vertiefung ist dazu vorzugsweise als Näpfchen ausgeführt.

In Fortführung kann der Boden des wannenförmigen Grundkörpers mehrere korrespondierend zu Behältnissen angeordnete Vertiefungen und die Vertiefungen verbindende Kanäle aufweisen, wobei die Barriere höher als die Kanalböden ist. Der Raum für Nährmedium ist damit weiter eingeschränkt, ohne dass die Versorgung der Objekte in den Behältnissen eingeschränkt ist.

Der plattenförmige Träger kann weiterhin wenigstens einen von einer Wand umgebenden Bereich aufweisen. Dieser besitzt bis auf einen Teilbereich einen Durchbruch. Der Teilbereich ist korrespondierend zu dem Septum angeordnet. Im Bereich befindet sich die Membran. Die Wand umschließt einen Raum, der der Aufnahme des Objekts dient. Damit ist eine eindeutige Platzierung des Objektes in diesem Raum möglich. Der Teilbereich dient vorteilhafterweise als Sperre für die das Objekt einbringende Vorrichtung beispielsweise in Form einer Kanüle. Damit wird das Einbringen des Objektes in das Medium im Grundkörper verhindert. Das Objekt ist damit einfach in die Einrichtung einbringbar.

Die Barriere ist nach der Weiterbildung des Patentanspruchs 5 eine beabstandet vor dem Auslass angeordnete Trennwand im wannenförmigen Grundkörper. Weiterhin sind der Einlass und der Auslass so angeordnet, dass die Höhe der Trennwand die Höhe der Oberfläche des Mediums im wannenförmigen Grundkörper bestimmt.

Vorteilhafterweise kann dabei wenigstens ein Bereich der Trennwand über eine Sollbruchstelle mit dem restlichen Bereich der Trennwand verbunden sein, so dass damit die Höhe des Mediums im Grundkörper beeinflussbar ist.

Barrieren sind nach der Weiterbildung des Patentanspruchs 6 sowohl eine beabstandet vor dem Auslass angeordnete erste Trennwand als auch eine beabstandet nach dem Einlass angeordnete zweite Trennwand im wannenförmigen Grundkörper. Darüber hinaus sind der Einlass und der Auslass so angeordnet, dass die Höhe der Trennwände die Höhe der Oberfläche des Mediums im wannenförmigen Grundkörper bestimmt.

Vorteilhafterweise kann dabei wenigstens ein Bereich wenigstens einer der Trennwände über eine Sollbruchstelle mit dem restlichen Bereich dieser Trennwand verbunden sein, so dass damit die Höhe des Mediums im Grundkörper beeinflussbar ist.

Die Barriere ist nach der Weiterbildung des Patentanspruchs 7 ein Bereich der Seitenwand mit dem Auslass des wannenförmigen Grundkörpers, wobei vorteilhafterweise der Abstand des Auslasses zum Boden des wannenförmigen Grundkörpers die Höhe der Barriere ist und dieser Abstand die Höhe der Oberfläche des Mediums im wannenförmigen Grundkörper bestimmt.

Der Boden des wannenförmigen Grundkörpers weist nach der Weiterbildung des Patentanspruchs 8 wenigstens eine die Strömung des Nährmediums beeinflussende Ausbuchtung und/oder Erhöhung auf, so dass Wirbel in der Strömung des Mediums vorhanden sind. Das Medium wird durch die Einrichtung transportiert, so dass immer frisches Medium über die Membran dem Objekt zugeführt wird.

Nach der Weiterbildung des Patentanspruchs 9 ist die Begasungseinheit vorteilhafterweise ein Oxygenator.

Der wannenförmige Basiskörper besitzt nach der Weiterbildung des Patentanspruchs 10 eine rechteckförmige Grundfläche. Die Fördereinrichtung und/oder wenigstens ein Steckverbinder für den Sensor und/oder ein Verbindungsmittel der Einrichtung zur Bestimmung wenigstens eines Stoffes des Mediums und/oder im Medium befindet oder befinden sich weiterhin an einer Wand des Basiskörpers oder sind Bestandteile dieser Wand. Darüber hinaus sind das Antriebsmittel korrespondierend zur Fördereinrichtung und/oder der wenigstens eine Steckverbinder korrespondierend zum Steckverbinder für den Sensor und/oder das Gegenstück korrespondierend zum Verbindungsmittel der Einrichtung zur Bestimmung wenigstens eines Stoffes des Mediums und/oder im Medium in einer Ebene der Aufnahmestation angeordnet. Mit der Platzierung des Basiskörpers auf der Aufnahmestation werden die jeweiligen mit Einrichtungen der Aufnahmestation zu verbindenden Bestandteile des Baukastensystems automatisch verbunden. Fehlverbindungen werden durch die jeweiligen festen Positionen der zu verbindenden Bestandteile des Basiskörpers und der Aufnahmestation vermieden. Damit ist ein sicherer Betrieb der durch den Baukasten realisierten Einrichtung zur Beeinflussung von biologischen Medien, Zellen, Gewebe oder gewebeähnlichen Strukturen als Objekte gewährleistet.

Vorteilhafterweise sind nach der Weiterbildung des Patentanspruchs 11 wenigstens der wannenförmige Basiskörper, der Behälter, die Fördereinrichtung, die Verbindungselemente, die Filter, der Bioreaktor und die Begasungseinheit als Bestandteile des Baukastensystems sterile Elemente des Baukastensystems. Das sind dann einfache Wegwerfprodukte.

Ein Ausführungsbeispiel der Erfindung ist in den Zeichnungen jeweils prinzipiell dargestellt und wird im Folgenden näher beschrieben.

Es zeigen:
Fig. 1 eine Einrichtung zur Beeinflussung von biologischen Medien, Zellen, Gewebe und gewebeähnlicher Strukturen als Objekte mit einem Bioreaktor und einem Oxygenator,
Fig. 2 einen Bioreaktor mit einem Bündel von Hohlfasermembranen,
Fig. 3 einen Bioreaktor zur Kultivierung von Zellen und Gewebekulturen sowie Hefen und Bakterien in einer Explosivdarstellung und
Fig. 4 eine Aufnahmestation für eine Einrichtung zur Beeinflussung von biologischen Medien, Zellen, Gewebe und gewebeähnlicher Strukturen als Objekte.

Ein bio- und medizintechnisches Baukastensystem zur Realisierung einer in sich geschlossenen Einrichtung zur Beeinflussung von biologischen Medien, Organismen oder Teilen von Organismen als Objekte besteht im Wesentlichen aus einem wannenförmigen Basiskörper 1 zur Platzierung von miteinander verbindbaren Elementen, wobei Behälter 2 für ein flüssiges Medium, eine Fördereinrichtung 3 für flüssiges Medium, Verbindungselemente, Filter 4 zur Be- und Entgasung, Filter 5 mit einem Anschlusselement für Medium, ein Bioreaktor 6 und eine Begasungseinheit in Form eines Oxygenators 7 Elemente und damit Bestandteile des Baukastensystems sind.

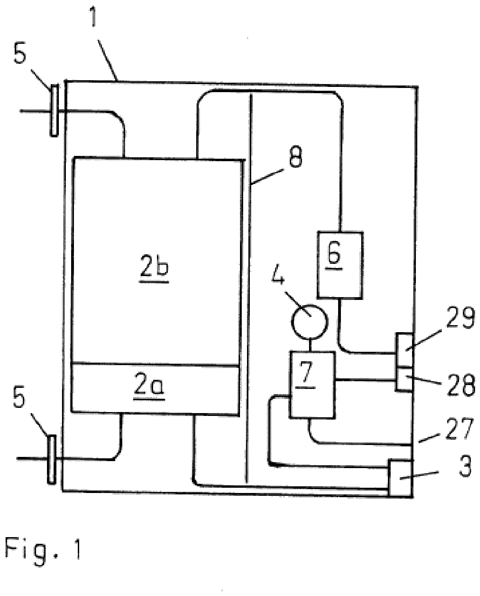

Die Fig. 1 zeigt eine Einrichtung zur Beeinflussung von biologischen Medien, Zellen, Gewebe und gewebeähnlicher Strukturen als Objekte mit einem Bioreaktor und einem Oxygenator in einer prinzipiellen Darstellung.

Der wannenförmige Basiskörper 1 weist eine rechteckförmige Grundfläche auf und besitzt wenigstens eine Zwischenwand 8, so dass ein Raum für die Behälter 2 für das flüssige Medium und ein Raum für den Bioreaktor 6 und den Oxygenator 7 vorhanden sind.

Die Behälter 2 sind bekannte Beutel 2 mit wenigstens jeweils einem Zu- und Abfluss für das flüssige Medium. Diese sind übereinander angeordnet, wobei der Beutel 2 mit Medium als erster Beutel 2a unter dem Beutel 2 für verbrauchtes Medium als zweiten Beutel 2b angeordnet ist. Wenigstens der erste Beutel 2a ist mit dem Filter 5 mit dem Anschlusselement verbunden, so dass dieser von außen mit Medium befüllbar ist.

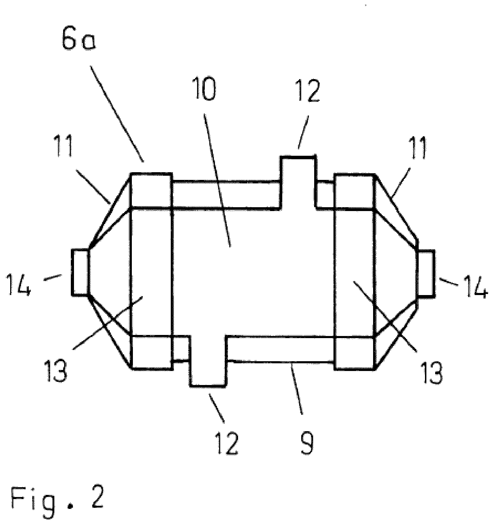

Die Fig. 2 zeigt einen Bioreaktor mit einem Bündel von Hohlfasermembranen in einer prinzipiellen Darstellung.

Ein erster Bioreaktor 6a besteht aus einem Gehäuse 9 mit einem Bündel 10 nach außen offener Hohlfasermembranen und kappenförmigen Endstücken 11. Das Gehäuse 9 ist ein Rohrabschnitt mit Anschlüssen 12 für den Raum mit den Hohlfasermembranen. Die Endenbereiche der Hohlfasermembranen befinden sich in Böden 13 aus einem Kunststoff. Die Endstücke 11 weisen jeweils einen Anschluss 14 für die Hohlräume des Raumes über den Enden der Hohlfasermembranen einschließlich der Böden 13 und der Hohlfasermembranen selbst auf. Das Bündel 10 von Hohlfasermembranen ist im Querschnitt rechteckförmig ausgebildet.

Ein Beutel 2 mit flüssigem Medium ist über die Fördereinrichtung 3 mit dem Oxygenator 7, dem Bioreaktor 6 und dem oder einem weiteren Behälter 2 für flüssiges Medium verbunden. Wenigstens einer der Beutel 2 ist mit dem Anschlusselement des schalenförmigen Basiskörpers 1 verbunden (Darstellungen der Fig. 1 und 2).

Ein zweiter Bioreaktor 6b zur Kultivierung von Zellen, Hefen und Bakterien besteht im Wesentlichen aus einem wannenförmigen Grundkörper 15, einem plattenförmigen Träger 18 und einem Deckel 19.

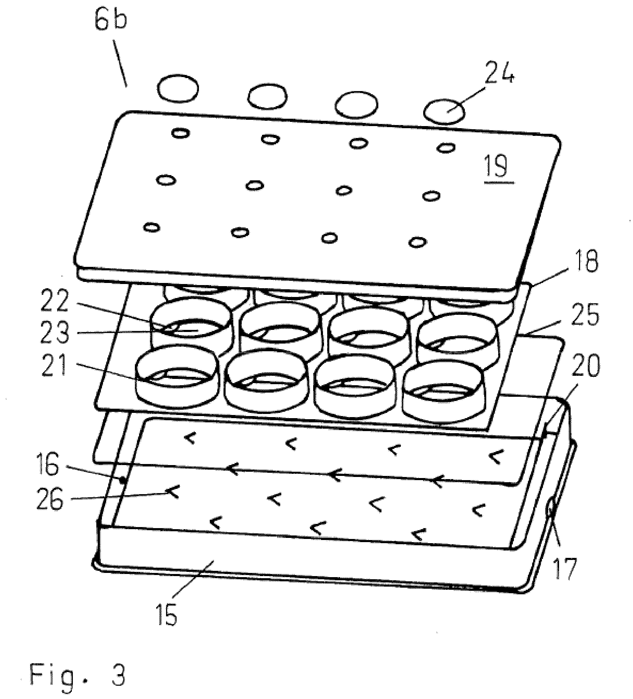

Die Fig. 3 zeigt den Bioreaktor 6b zur Kultivierung von Zellen und Gewebekulturen sowie Hefen und Bakterien in einer prinzipiellen Explosionsdarstellung.

Der wannenförmige Grundkörper 15 besitzt eine rechteckförmige Grundfläche. Ein Einlass 16 und ein Auslass 17 für das Nährmedium befinden sich an gegenüberliegenden Seitenwänden des wannenförmigen Grundkörpers 15. Beabstandet vor dem Auslass 17 ist eine Trennwand 20 angeordnet.

Der plattenförmige Träger 18 befindet sich im wannenförmigen Grundkörper 15. Dieser weist von Wänden 21 umgebende Bereiche auf, die jeweils bis auf einen Teilbereich 22 einen Durchbruch 23 besitzen. Weiterhin befindet sich im Bereich die Membran, die in der Fig. 3 nicht dargestellt ist. Die Membranen und die Wände 21 stellen damit ein Behältnis für das Objekt dar.

Die Membranen aufweisenden Behältnisse sind im Raum zwischen der Seitenwand mit dem Einlass 16 und der Trennwand 20 angeordnet. Die Behältnisse können darüber hinaus auch einzeln platzierte Behältnisse oder Bestandteile des plattenförmigen Trägers 18 sein.

Der Einlass 16 und der Auslass 17 sind so angeordnet, dass die Höhe der Trennwand 20 die Höhe der Oberfläche des Nährmediums im wannenförmigen Grundkörper 15 bestimmt, wobei sich wenigstens die Membranen der Behältnisse im Nährmedium des wannenförmigen Grundkörpers 15 befinden. Dazu sind der Einlass 16 und der Auslass 17 tiefer als die Trennwand 20 hoch ist angeordnet.

Der Deckel 19 ist mit Septen 24 ausgestattet. Die Teilbereiche 22 sind korrespondierend zu den Septen 24 angeordnet.

Der wannenförmige Grundkörper 15 und der Deckel 19 sind lösbar dicht miteinander verbunden. Das wird beispielsweise über mindestens eine umlaufende O-Ringdichtung 25 zwischen Deckel 19 und wannenförmigen Grundkörper 15 gewährleistet.

Der Boden des wannenförmigen Grundkörpers 15 weist in einer weiteren Ausführungsform des zweiten Bioreaktors 6b wenigstens eine die Strömung des Nährmediums beeinflussende Erhöhung 26 auf, so dass Wirbel in der Strömung des Nährmediums vorhanden sind.

In einer weiteren Ausführungsform des zweiten Bioreaktors 6b besitzt der Boden des wannenförmigen Grundkörpers 15 korrespondierend zu den Behältnissen angeordnete Vertiefungen, so dass zwischen den Behältnissen für die Objekte und den Vertiefungen ein Abstand für das Nährmedium vorhanden ist. Weiterhin weist der Boden des wannenförmigen Grundkörpers 15 die Vertiefungen verbindende Kanäle auf. Die Trennwand 20 ist dabei höher als die Ebene des Bodens mit den Vertiefungen und höher als die Böden der Kanäle.

Wenigstens ein Bereich der Trennwand 20 ist in einer weiteren Ausführungsform des zweiten Bioreaktors 6b über eine Sollbruchstelle mit dem restlichen Bereich der Trennwand 20 verbunden.

Der Oxygenator 7 ist ein Behältnis mit Anschlüssen, so dass Medium im Behälter mit Sauerstoff anreicherbar ist. Der Sauerstoff wird von außen zugeführt, wozu dafür der wannenförmige Basiskörper 1 ein Anschlusselement 27 besitzt, welches über ein Schlauchstück als Verbindungselement mit dem Oxygenator 7 verbunden ist.

Nach Darstellung in Fig. 1 ist der erste Behälter 2a über die Fördereinrichtung 3 in Form einer bekannten Pumpe 3 und Schlauchstücke als Verbindungselemente mit dem Oxygenator 7, dem Bioreaktor 6 und dem zweiten Beutel 2b als zweiten Behälter 2b verbunden. Die Fördereinrichtung 3 ist dazu ein Bestandteil der Wand des wannenförmigen Basiskörpers 1 oder befindet sich im Raum mit dem Oxygenator 7 und dem Bioreaktor 6. Damit ist das Antriebsmittel der Fördereinrichtung 3 oder die Energiequelle für das Antriebsmittel der Fördereinrichtung 3 von außen lösbar anschließbar. Im ersteren Fall besteht die Pumpe aus der Fördereinrichtung 3 und dem Antriebsmittel, die lösbar miteinander gekoppelt sind. Das Antriebsmittel ist dazu ein bekannter Elektromotor mit einem Rotor, der formschlüssig mit der Fördereinrichtung 3 verbindbar ist. Dazu kann der Rotor wenigstens bereichsweise im Querschnitt mehreckig oder sternförmig ausgebildet sein oder wenigstens eine ebene Fläche besitzen.

An einer Wand des wannenförmigen Basiskörpers 1 ist weiterhin ein mit dem Oxygenator 7 verbundener pH-Wert-Sensor 28 und/oder Sauerstoffsensor 29 angeordnet. Letzterer wird von außerhalb des wannenförmigen Basiskörpers 1 optisch oder elektrisch angekoppelt.

Zwischen dem Oxygenator 7 und dem Bioreaktor 6 kann sich somit ein erster Sauerstoffsensor 29 und nach dem Bioreaktor 6 ein zweiter Sauerstoffsensor und der pH-Wert-Sensor 28 befinden.

Der Basiskörper 1 ist dazu vorteilhafterweise so ausgebildet, dass die Anschlüsse für den Sensor 28, 29 und/oder eine Einrichtung zur Bestimmung wenigstens eines Stoffes des Mediums und/oder im Medium an einer Wand des Basiskörpers 1 angeordnet oder Bestandteile dieser Wand sind.

Die einzelnen Verbindungen sind durch gerade Rohrstücke, gebogene Rohrstücke, Schlauchstücke, Verbinder und/oder Verteiler gebildet. Gleichzeitig sind Rückschlagventile so angeordnet, dass auch ein Medienfluss ohne die Beutel 2 realisierbar ist. Die Umkehrung des Medienflusses wird realisiert in dem entweder die Förderrichtung der Fördereinrichtung 3 umgekehrt wird oder eine zusätzliche Fördereinrichtung 3 mit umgekehrter Flussrichtung integriert wird.

Die Einrichtung ist damit vorteilhafterweise gleichzeitig ein horizontaler Einschub, wobei die Fördereinrichtung 3 und der wenigstens eine Sensor im platzierten Zustand kontaktiert und damit betriebsbereit sind. Die Anschlusselemente des wannenförmigen Basiskörpers 1 sind von vorn frei zugänglich, so dass das flüssige Medium einfach zuführbar ist. Der wannenförmige Basiskörper 1 ist bei einer derartigen Betriebsweise der Einrichtung vorteilhafterweise gleichzeitig ein Schutz vor Auslaufen des flüssigen Mediums. Bei Funktionsstörungen durch Leckagen verbleibt das flüssige Medium im wannenförmigen Basiskörper 1. Damit können mehrere derartige Einrichtungen einfach auch übereinander angeordnet werden.

Die Einrichtung selbst ist ökonomisch günstig realisiert, so dass nach der Nutzung die Einrichtung auch einfach entsorgbar ist.

In einer weiteren Ausführungsform der Einrichtung ist eine Aufnahmestation 30 für den wannenförmigen Basiskörper 1 ein Bestandteil für das Baukastensystem.

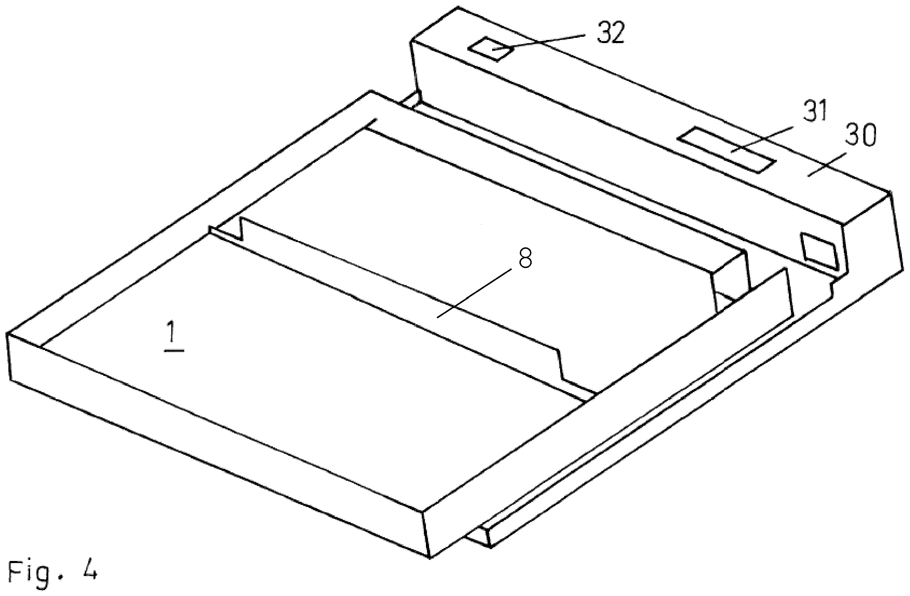

Die Fig. 4 zeigt eine Aufnahmestation 30 für eine Einrichtung zur Beeinflussung von biologischen Medien, Zellen, Gewebe und gewebeähnlicher Strukturen als Objekte in einer prinzipiellen Darstellung.

Bei Platzierung des wannenförmigen Basiskörpers 1 in der Aufnahmestation 30 ist das Antriebsmittel und/oder die Energiequelle für das Antriebsmittel mit der Fördereinrichtung 3 automatisch lösbar verbunden. Die Aufnahmestation 30 weist eine Steuereinrichtung in Form eines bekannten Datenverarbeitungssystems als Mikrocontroller für das Antriebsmittel der Fördereinrichtung 3 und die Signale der Sensoren 28, 29 auf. Mittels einer entsprechenden Software kann damit die Beeinflussung von biologischen Medien, Organismen oder Teilen von Organismen als Objekte gezielt gesteuert werden.

Der Betriebszustand ist durch eine mit der Steuereinrichtung verbundene Anzeigeeinheit 31 darstellbar.

Die Aufnahmestation 30 oder mehrere Aufnahmestationen 30 sind über ein externes lösbares Netzteil mit einem elektrischen Netz verbindbar. Für den Betrieb ist ein elektrischer Schalter 32 vorgesehen.

## Patentansprüche

1. Bio- und medizintechnisches Baukastensystem umfassend eine in sich geschlossenen Einrichtung zur Beeinflussung von biologischen Medien, Zellen, Gewebe und gewebeähnlicher Strukturen als Objekte mit einem wannenförmigen Basiskörper (1) zur Platzierung von miteinander verbindbaren Elementen, wobei wenigstens ein Behälter (2) für ein flüssiges Medium, wenigstens eine Fördereinrichtung (3) für flüssiges Medium an einer Wand am oder im wannenförmigen Basiskörper (1) oder als Bestandteil der Wand des Basiskörpers (1), so dass das Antriebsmittel oder die Energiequelle für das Antriebsmittel der Fördereinrichtung (3) von außen lösbar anschließbar ist, Verbindungselemente, Filter zur Be- und Entgasung (4), mindestens ein Filter (5) mit einem Anschlusselement für Medium und wenigstens ein Sensor (28, 29) und/oder eine Einrichtung zur Bestimmung wenigstens eines Stoffes des Mediums und/oder im Medium, Elemente für eine Verbindung mit wenigstens einem Bioreaktor (6) und/oder mindestens einer Begasungseinheit sowie ein Bereich für den Bioreaktor (6) und/oder die Begasungseinheit und ein Bereich für den Behälter (2) mit dem flüssigen Medium, wobei die Bereiche durch wenigstens eine Zwischenwand voneinander getrennt sind, vorhanden sind, und einer Aufnahmestation (30) für den wannenförmigen Basiskörper (1) mit einer Steuereinrichtung, wobei bei Platzierung des wannenförmigen Basiskörpers (1) in der Aufnahmestation (30) zum Einen das Antriebsmittel oder die Energiequelle für das Antriebsmittel mit der Fördereinrichtung (3) und zum Anderen das Antriebsmittel und entweder der Sensor (28, 29) und/oder die Einrichtung zur Bestimmung wenigstens eines Stoffes des Mediums und/oder im Medium mit der Steuereinrichtung verbunden sind.

2. Baukastensystem nach Patentanspruch 1, **dadurch gekennzeichnet, dass** der Bioreaktor (6) ein Gehäuse (9) mit einem nach außen offenen Bündel (10) von Hohlfasermembranen und kappenförmigen Endstücken (11) aufweist, dass das Gehäuse (9) ein Rohrabschnitt mit Anschlüssen (12) für den Raum mit den Hohlfasermembranen ist, dass sich die Endenbereiche der Hohlfasermembranen in Böden (13) aus einem Kunststoff befinden und dass die Endstücke (11) jeweils einen Anschluss (14) für die Hohlräume der Hohlfasermembranen aufweisen.

3. Baukastensystem nach Patentanspruch 1, **dadurch gekennzeichnet, dass** der Bioreaktor (6) einen wannenförmigen Grundkörper (15) mit jeweils wenigstens einem Einlass (16) und einem Auslass (17) in einer der Seitenwände für das Medium und einen von dem wannenförmigen Grundkörper (15) entfernbaren Deckel (19) ohne oder mit mindestens einem Septum (24) aufweist.

4. Baukastensystem nach Patentanspruch 1, **dadurch gekennzeichnet, dass** der Bioreaktor (6) einen wannenförmigen Grundkörper (15) mit jeweils wenigstens einem Einlass (16) und einem Auslass (17) in einer der Seitenwände für das Medium, mindestens eine waagerechte Barriere im wannenförmigen Grundkörper (15) oder als Bestandteil des wannenförmigen Grundkörpers (15), einen plattenförmigen Träger (18) mit mindestens einem eine Membran aufweisenden Behältnis für das wenigstens eine Objekt in oder als Bestandteil des plattenförmigen Trägers (18) im wannenförmigen Grundkörper (15) und einen von dem wannenförmigen Grundkörper (15) entfernbaren Deckel (19) mit mindestens einem Septum (24) aufweist, wobei der Einlass (16) und der Auslass (17) so angeordnet sind, dass die Höhe der Barriere die Höhe der Oberfläche des Mediums im wannenförmigen Grundkörper (15) bestimmt, und wobei sich wenigstens die Membran des Behältnisses im Medium des wannenförmigen Grundkörpers (15) befindet.

5. Baukastensystem nach Patentanspruch 4, **dadurch gekennzeichnet, dass** die Barriere eine beabstandet vor dem Auslass (17) angeordnete Trennwand (20) im wannenförmigen Grundkörper (15) ist und dass der Einlass (16) und der Auslass (17) so angeordnet sind, dass die Höhe der Trennwand (20) die Höhe der Oberfläche des Mediums im wannenförmigen Grundkörper (15) bestimmt.

6. Baukastensystem nach Patentanspruch 4, **dadurch gekennzeichnet, dass** Barrieren sowohl eine beabstandet vor dem Auslass (17) angeordnete erste Trennwand (20) als auch eine beabstandet nach dem Einlass angeordnete zweite Trennwand (20) im wannenförmigen Grundkörper (15) sind und dass der Einlass (16) und der Auslass (17) so angeordnet sind, dass die Höhe der Trennwände (20) die Höhe der Oberfläche des Mediums im wannenförmigen Grundkörper (15) bestimmt.

7. Baukastensystem nach jeweils einem der Patentansprüche 4 bis 6, **dadurch gekennzeichnet, dass** die Barriere ein Bereich der Seitenwand mit dem Auslass (17) des wannenförmigen Grundkörpers (15) ist, wobei der Abstand des Auslasses (17) zum Boden des wannenförmigen Grundkörpers (15) die Höhe der Barriere ist und dieser Abstand die Höhe der Oberfläche des Mediums im wannenförmigen Grundkörper (15) bestimmt.

8. Baukastensystem nach Patentanspruch 4, **dadurch gekennzeichnet, dass** der Boden des wannenförmigen Grundkörpers (15) wenigstens eine die Strömung des Nährmediums beeinflussende Ausbuchtung und/oder Erhöhung (26) aufweist, so dass Wirbel in der Strömung des Mediums vorhanden sind.

9. Baukastensystem nach Patentanspruch 1, **dadurch gekennzeichnet, dass** die Begasungseinheit ein Oxygenator (7) ist.

10. Baukastensystem nach Patentanspruch 1, **dadurch gekennzeichnet, dass** der wannenförmige Basiskörper (1) eine rechteckförmige Grundfläche besitzt,
dass sich die Fördereinrichtung (3) und/oder wenigstens ein Steckverbinder für den Sensor (28, 29) und/oder ein Verbindungsmittel der Einrichtung zur Bestimmung wenigstens eines Stoffes des Mediums und/oder im Medium an einer Wand des Basiskörpers (1) befinden und
dass das Antriebsmittel korrespondierend zur Fördereinrichtung (3) und/oder der wenigstens eine Steckverbinder korrespondierend zum Steckverbinder für den Sensor (28, 29) und/oder das Gegenstück korrespondierend zum Verbindungsmittel der Einrichtung zur Bestimmung wenigstens eines Stoffes des Mediums und/oder im Medium in einer Ebene der Aufnahmestation (30) angeordnet sind.

11. Baukastensystem nach Patentanspruch 1, **dadurch gekennzeichnet, dass** wenigstens der wannenförmige Basiskörper (1), der Behälter (2), die Fördereinrichtung (3), die Verbindungselemente, die Filter (4, 5), der Bioreaktor (6) und die Begasungseinheit als Bestandteile des Baukastensystems sterile Elemente des Baukastensystems sind.

## Claims

1. Bioengineering and medical modular system comprising a self-contained equipment for influencing biological media, cells, tissue and tissue-like structures as objects with a tub-shaped main body (1) for placement of elements that can be combined with one another, wherein there are at least one container (2) for a liquid medium, at least one pumping system (3) for the liquid medium on a wall on or in the tub-shaped main body (1) or as a component part of the wall of the main body (1), so that a drive unit or an energy source for the drive unit of the pumping system (3) can be connected from the outside so as to be detachable, connection elements, filters for the inlet and outlet of gas (4), at least one filter (5) with a connecting element for the medium and at least one sensor (28, 29) and/or a device for identifying at least one substance of the medium and/or in the medium, elements for a connection with at least one bioreactor (6) and/or at least one gas inlet unit, as well as an area for at least one bioreactor (6) or at least one gas inlet unit and an area for the container (2) for the liquid medium, wherein the areas are separated from one another by at least one partition wall, and a seating station (30) for the tub-shaped main body (1) with a control unit, wherein the tub-shaped main body (1) is placed in the seating station (30) such, that the drive unit or the energy source for the drive unit is connected to the pumping system (3) and the drive unit and either the sensor (28, 29) and/or the device for identifying at least one substance of the medium and/or in the medium is connected to the control unit.

2. Modular system according to claim 1, **characterized in that** the bioreactor (6) has a housing (9) with a bundle (10) of hollow-fiber membranes opening towards the outside and cap-shaped end pieces (11), that the housing (9) is a pipe section with connectors (12) for the space with the hollow-fiber membranes, that end areas of the hollow-fiber membranes are in bases (13) made of plastic and that the end pieces (11) have a connector (14) in each case for the hollow spaces of the hollow-fiber membranes.

3. Modular system according to claim 1, **characterized in that** the bioreactor (6) has a tub-shaped base body (15) with in each case at least one inlet (16) and an outlet (17) in one of the side walls for the medium and a cover (19) without or with at least one septum (24), said cover being capable of being removed from the tub-shaped base body (15).

4. Modular system according to claim 1, **characterized in that** the bioreactor (6) has a tub-shaped base body (15) with in each case at least one inlet (16) and an outlet (17) in one of the side walls for the medium, at least one horizontal barrier in the tub-shaped base body (15) or as a component part of the tub-shaped base body (15), a plate-shaped holder (18) with at least one receptacle having a membrane for the at least one object in or as a component part of the plate-shaped holder (18) in the tub-shaped base body (15), and a cover (19) with at least one septum (24), said cover being capable of being removed from the tub-shaped base body (15), wherein the inlet (16) and the outlet (17) are arranged in such a way that the height of the barrier determines the height of the surface of the medium in the tub-shaped base body (15) and wherein at least the membrane of the receptacle is located in the medium of the tub-shaped base body (15).

5. Modular system according to claim 4, **characterized in that** the barrier is a partition wall (20) arranged at a distance in front of the outlet (17) in the tub-shaped base body (15) and that the inlet (16) and the outlet (17) are arranged in such a way that the height of the partition wall (20) determines the height of the surface of the medium in the tub-shaped base body (15).

6. Modular system according to claim 4, **characterized in that** both a first partition wall (20) arranged at a distance in front of the outlet (17) and a second partition wall (20) arranged at a distance after the inlet are barriers in the tub-shaped base body (15) and that the inlet (16) and the outlet (17) are arranged in such a way that the height of the partition walls (20) determines the height of the surface of the medium in the tub-shaped base body (15).

7. Modular system according to one of the claims 4 to 6 in each case, **characterized in that** the barrier is an area of the side wall with the outlet (17) of the tub-shaped base body (15), wherein the distance of the outlet (17) to the floor of the tub-shaped base body (15) is the height of the barrier and this distance determines the height of the surface of the medium in the tub-shaped base body (15).

8. Modular system according to claim 4, **characterized in that** the floor of the tub-shaped base body (15) has at least one protrusion and/or elevation (26) influencing the flow of a culture medium so that vortices exist in the flow of the medium.

9. Modular system according to claim 1, **characterized in that** the gas inlet unit is an oxygenator (7).

10. Modular system according to claim 1, **characterized in that** the tub-shaped main body (1) has a rectangular base surface,
that the pumping system (3) and/or at least one plug-type connector for the sensor (28, 29) and/or a connecting unit of the device for identifying at least one substance of the medium and/or in the medium is located on a wall of the main body (1) and
that the drive unit corresponding to the pumping system (3) and/or at least one plug-type connector corresponding to the plug-type connector for the sensor (28, 29) and/or the counterpart corresponding to the connecting unit of the device for identifying at least one substance of the medium and/or in the medium is arranged in the plane of the seating station (30).

11. Modular system according to claim 1, **characterized in that** at least the tub-shaped main body (1), the container (2), the pumping system (3), the connection elements, the filters (4, 5), the bioreactor (6) and the gas inlet unit, as component parts of the modular system, are sterile elements of the modular system.

## Revendications

1. Système modulaire biotechnologique et médical comprenant un dispositif fermé en soi pour influencer des milieux, cellules et tissus biologiques et des structures analogues à des tissus en tant qu'objets comportant un corps de base (1) en forme de bac pour le positionnement d'éléments pouvant être reliés entre eux, au moins un récipient (2) pour un milieu liquide, au moins un dispositif d'acheminement (3) pour un milieu liquide sur une paroi sur ou dans le corps de base (1) en forme de bac ou en tant que composant de la paroi du corps de base (1), de sorte que le moyen d'entraînement ou la source d'énergie pour le moyen d'entraînement du dispositif d'acheminement (3) peut être raccordé de manière amovible depuis l'extérieur, des éléments de liaison, des filtres pour le gazage et le dégazage (4), au moins un filtre (5) comportant un élément de raccordement pour un milieu et au moins un capteur (28, 29) et/ou un dispositif pour déterminer au moins une substance du milieu et/ou dans le milieu, des éléments pour une liaison avec au moins un bioréacteur (6) et/ou au moins une unité de gazage ainsi qu'une zone pour le bioréacteur (6) et/ou l'unité de gazage et une zone pour le contenant (2) avec le milieu liquide, les zones étant séparées les unes des autres par au moins une paroi intermédiaire, étant présents, et une station de réception (30) pour le corps de base (1) en forme de bac comportant un dispositif de commande, lors du positionnement du corps de base (1) en forme de bac dans la station de réception (30) d'une part le moyen d'entraînement ou la source d'énergie pour le moyen d'entraînement étant relié au dispositif d'acheminement (3) et d'autre part le moyen d'entraînement et le capteur (28, 29) et/ou le dispositif pour déterminer au moins une substance du milieu et/ou dans le milieu étant reliés au dispositif de commande.

2. Système modulaire selon la revendication 1, **caractérisé en ce que** le bioréacteur (6) comporte un boîtier (9) pourvu d'un ensemble (10) ouvert vers l'extérieur de membranes à fibres creuses et d'embouts (11) en forme de calotte, **en ce que** le boîtier (9) est une portion tubulaire comportant des raccords (12) pour le compartiment comportant les membranes à fibres creuses, **en ce que** les zones d'extrémité des membranes à fibres creuses se trouvent dans des parties inférieures (13) en matière plastique et **en ce que** les embouts (11) comportent chacun un raccord (14) pour les cavités des membranes à fibres creuses.

3. Système modulaire selon la revendication 1, **caractérisé en ce que** le bioréacteur (6) comporte un corps de base (15) en forme de bac comportant au moins une admission (16) et une évacuation (17) dans une des parois latérales pour le milieu et un couvercle (19) pouvant être retiré du corps de base (15) en forme de bac sans ou avec au moins une cloison (24).

4. Système modulaire selon la revendication 1, **caractérisé en ce que** le bioréacteur (6) comporte un corps de base (15) en forme de bac comportant au moins une admission (16) et une évacuation (17) dans une des parois latérales pour le milieu, au moins une barrière horizontale dans le corps de base (15) en forme de bac ou en tant que composant du corps de base (15) en forme de bac, un support (18) en forme de plaque comportant au moins un récipient pourvu d'une membrane pour l'au moins un objet dans ou en tant que composant du support (18) en forme de plaque dans le corps de base (15) en forme de bac et un couvercle (19) pouvant être retiré du corps de base (15) en forme de bac et comportant au moins une cloison (24), l'admission (16) et l'évacuation (17) étant disposées de sorte que la hauteur de la barrière détermine la hauteur de la surface du milieu dans le corps de base (15) en forme de bac, et au moins la membrane du récipient se trouvant dans le milieu du corps de base (15) en forme de bac.

5. Système modulaire selon la revendication 4, **caractérisé en ce que** la barrière est une paroi de séparation (20), disposée à distance avant l'évacuation (17), dans le corps de base (15) en forme de bac et **en ce que** l'admission (16) et l'évacuation (17) sont disposées de sorte que la hauteur de la paroi de séparation (20) détermine la hauteur de la surface du milieu dans le corps de base (15) en forme de bac.

6. Système modulaire selon la revendication 4, **caractérisé en ce que** des barrières sont aussi bien une première paroi de séparation (20) disposée à distance avant l'évacuation (17) qu'une seconde paroi de séparation (20) disposée à distance après l'admission, dans le corps de base (15) en forme de bac et **en ce que** l'admission (16) et l'évacuation (17) sont disposées de sorte que la hauteur des parois de séparation (20) détermine la hauteur de la surface du milieu dans le corps de base (15) en forme de bac.

7. Système modulaire selon respectivement l'une des revendications 4 à 6, **caractérisé en ce que** la barrière est une zone de la paroi latérale comportant l'évacuation (17) du corps de base (15) en forme de bac, la distance de l'évacuation (17) à la partie inférieure du corps de base (15) en forme de bac étant la hauteur de la barrière et cette distance déterminant la hauteur de la surface du milieu dans le corps de base (15) en forme de bac.

8. Système modulaire selon la revendication 4, **caractérisé en ce que** la partie inférieure du corps de base (15) en forme de bac comporte au moins un bombement et/ou une élévation (26) influençant l'écoulement du milieu nutritif, de sorte que des tourbillons sont présents dans l'écoulement du milieu.

9. Système modulaire selon la revendication 1, **caractérisé en ce que** l'unité de gazage est un oxygénateur (7).

10. Système modulaire selon la revendication 1, **caractérisé en ce que** le corps de base (1) en forme de bac possède une surface de base rectangulaire,
**en ce que** le dispositif d'acheminement (3) et/ou au moins un connecteur enfichable pour le capteur (28, 29) et/ou un moyen de liaison du dispositif pour déterminer au moins une substance du milieu et/ou dans le milieu se trouvent sur une paroi du corps de base (1) et
**en ce que** le moyen d'entraînement correspondant au dispositif d'acheminement (3) et/ou l'au moins un connecteur enfichable correspondant au connecteur enfichable pour le capteur (28, 29) et/ou la pièce homologue correspondant au moyen de liaison du dispositif pour déterminer au moins une substance du milieu et/ou dans le milieu sont disposés dans un plan de la station de réception (30).

11. Système modulaire selon la revendication 1, **caractérisé en ce qu'**au moins le corps de base (1) en forme de bac, le récipient (2), le dispositif d'acheminement (3), les éléments de liaison, les filtres (4, 5), le bioréacteur (6) et l'unité de gazage en tant que composants du système modulaire sont des éléments stériles du système modulaire.
